(19) Europäisches Patentamt
European Patent Office
Office européen des brevets

(11) Veröffentlichungsnummer: **0 074 103**
**B1**

# (12) EUROPÄISCHE PATENTSCHRIFT

(45) Veröffentlichungstag der Patentschrift: **29.01.86**

(21) Anmeldenummer: **82108166.8**

(22) Anmeldetag: **03.09.82**

(51) Int. Cl.[4]: **C 07 K 5/04**, C 07 K 5/06, C 07 C 127/17, C 07 C 127/15, C 07 C 111/00

## (54) N-(N'-(2-Chloräthyl)-N'-nitroso-carbamoyl)- oligopeptidester und -amide und Verfahren zu ihrer Herstellung.

(30) Priorität: **03.09.81 DE 3134923**

(43) Veröffentlichungstag der Anmeldung: **16.03.83 Patentblatt 83/11**

(45) Bekanntmachung des Hinweises auf die Patenterteilung: **29.01.86 Patentblatt 86/5**

(84) Benannte Vertragsstaaten: **AT BE CH DE FR GB IT LI LU NL SE**

(56) Entgegenhaltungen:
**ARZNEIMITTEL FORSCHUNG DRUG RESEARCH, Band 32 (I), Nr. 5, Mai 1982, Seiten 484-486, Aulendorf (DE); W.J. ZELLER et al.: "Chemotherapeutic activity of 2-chloroethylnitrosocarbamoyl derivatives of amino acids in a transplanted rat leukemia (L5222)".**
**CHEMICAL ABSTRACTS, Band 92, Nr. 3, 21. Januar 1980, Seite 54, Nr. 15507u, Columbus Ohio (USA); W.J. ZELLER et al.: "*Examination of four newly synthesized 2-chloroethylnitrosoureas in comparison with BCNU, CCNU, MeCCNU, chlorozoticin and hydroxyethyl-CNU in preterminal rat leukemia L 5222*".**
**CHEMICAL ABSTRACTS, Band 85, Nr. 1, 5. Juli 1976, Seiten 491-492, Nr. 6050t, Columbus Ohio (USA);**

**Die Akte enthält technische Angaben, die nach dem Eingang der Anmeldung eingereicht wurden und die nicht in dieser Patentschrift enthalten sind.**

(73) Patentinhaber: **Stiftung Deutsches Krebsforschungszentrum, Im Neuenheimer Feld 280, D-6900 Heidelberg 1 (DE)**

(72) Erfinder: **Eisenbrand, Gerhard, Dr.,** ***Banngartenstrasse 19, D-6902 Sandhausen (DE)***
Erfinder: **Tang, Wei-ci, Prof., Research Institute for Medicine and Pharmacy, Lai-wu Street II, No. 2a Quing dao (CN)**
Erfinder: **Zelesny, Otto, Emmertsgrundpassage 17, D-6900 Heidelberg (DE)**

(74) Vertreter: **Patentanwälte Müller-Boré, Deufel, Schön, Hertel, Lewald, Otto, Postfach 26 02 47 Isartorplatz 6, D-8000 München 26 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 074 103 B1

## Beschreibung

Unter den Alkylantien ist in den letzten Jahren eine neue Stoffklasse in die Krebschemotherapie eingeführt worden, nämlich die Nitrosoharnstoffe, z.B. 1,3-bis- (2-Chloräthyl)- 1-nitrosoharnstoff (BCNU), 1-(2-Chloräthyl)-3-cyclohexyl-1-nitrosoharnstoff (CCNU) und 1-(2-Chloräthyl) -3- (4-methylcyclohexyl) -1-nitrosoharnstoff (MeCCNU) (Proceeding of the 7th new drug seminar on the nitroso-ureas (Washington, D.C., Dec. 15–16, 1975), Cancer Treat. Rep., 60, 645–811 (1976)). Um Substanzen mit besserem therapeutischem Index zu erhalten, hat man in der letzten Zeit eine Reihe neuer Nitrosoharnstoffe mit verschiedenen Substituenten, z.B. Zuckerderivate, synthetisiert und experimentell getestet. (Anderson et al., Cancer Res. 35, 761 (1976), Hayat et al., Cancer Chemother. Pharmacol. 3, 217 (1979.))

Im Gegensatz zu den bisher synthetisierten Substanzen handelt es sich im vorliegenden Fall um Oligopeptidester, die an der Aminogruppe eine N-(2-Chloräthyl)- N-nitroso-carbamoylgruppierung tragen. Als Ausgangsmaterial zur Synthese von N-[N'(2-Chloräthyl)- N'-nitrosocarbamoyl],- oligopeptidestern dienen N[N'-(2-Chloräthyl) N'-nitroso-carbamoyl] aminosäuren oder -oligopeptide, die ihrerseits zweckmässig durch Reaktion von N-(2-Chloräthyl)- N-nitrosocarbamoylazid mit Aminosäuren bzw. Oligopeptiden zugänglich sind. Unter dem Einfluss von an sich bekannten Peptidkatalysatoren wie z.B. Dicyclohexylcarbodiimid (DCC) reagieren die erwähnten N[N'-(2-Chloräthyl)- N'-nitroso- carbamoyl] aminosäuren bzw. -oligopeptide zu Amiden, so dass sie mit dem Ester oder Amid einer zweiten Aminosäure bzw. eines zweiten Oligopeptids eine Peptidbindung bilden. Die Reaktion kann schematisch wie folgt dargestellt werden:

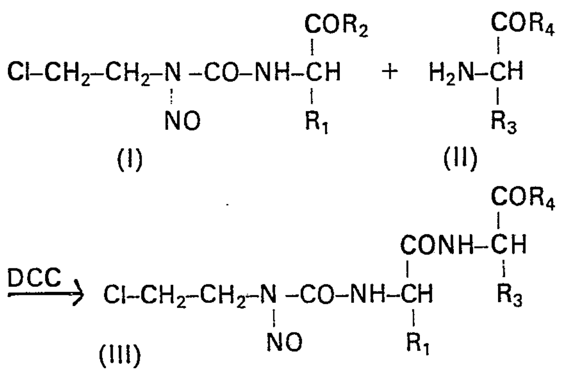

(I) (II) (III)

$R_1 = R_3 = -H, -CH_3, -CH(CH_3)_2, -CH_2-CH(CH_3)_2,$

$-CH(CH_3)C_2H_5, -C_2H_5, -CH_2$ 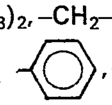 $, -CH_2OH,$

$-CH(OH)CH_3, -CH_2-CH_2-S-CH_3,$

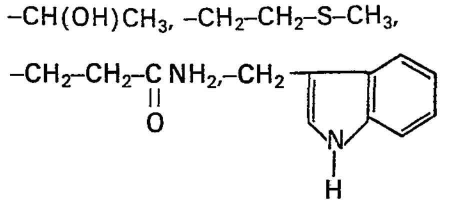

$R_2 = OH$ bzw. $-NH-R_2'$, wobei $R_2'$ einen über die terminale Aminogruppe peptidisch gebundenen Oligopeptidrest mit freier Carboxylgruppe darstellt.

Beispiele für $R_2'$:
- Glycyl-Glycin
- Glycyl-Glycyl-Glycin und Peptide anderer Aminosäuren bis zum Tripeptid, sowie gemischt Oligopeptide wie:

Glycyl-Alanin
Alanyl-Leucin
Leucyl-Alanin
Glycyl-Tyrosin
Leucyl-Glycyl-Tyrosin
Leucyl-Glycyl-Prolin

$R_4 = OCH_3, NH_2, -NHR_4'$, wobei $R_4'$ Alkyl oder Aryl bzw. einen über die terminale Aminogruppe peptidisch gebundenen Oligopeptidrest darstellt, dessen endständige Carboxylgruppe ihrerseits als Methylester oder Amid vorliegt.

Beispiele für $R_4'$:
-Glycin-Methylester
-Glycyl-Glycyl-Methylester
-Glycyl-Glycyl-Glycylmethylester

Peptide anderer Aminosäuren bis Tripeptid sowie gemischte Oligopeptide wie:
Glycyl-Alanin-Methylester
Leucyl-Glycyl-Prolinmethylester
Leucyl-Alanin-Amid
Glycyl-Tyrosin-Amid

Die Umsetzung des Ausgangsmaterials I mit dem Ester bzw. Amid einer weiteren Aminsäure oder eines Oligopeptids II, die gleich oder verschieden von der ersten Aminosäure bzw. dem ersten Oligopeptid sein können, erfolgt vorzugsweise unter Kühlung (etwa 0 °C), aber auch problemlos bei Raumtemperatur. Beispielsweise werden 1 mM N-[N'- (2-Chloräthyl)- N'-nitroso-carbamoyl]- aminosäure und 1 mM Aminosäuremethylester zusammen in 20 ml Dichlormethan gelöst. In diese Lösung wird unter Rühren 1,1 mM (230 mg) Dicyclheyl-carbodiimid, gelöst in 10 ml Dichlormethan, langsam zugetropft. Das Reaktionsgemisch wird dann weiter eine Stunde gerührt und über Nacht bei Zimmertemperatur stehengelassen. Nach Abfiltrieren von N,N'-Dicyclohexylharnstoff wird das Filtrat unter Vakuum eingeengt. Man erhält das Rohprodukt, das man säulenchromatographisch mit Ether/Dichlormethan unterschiedlicher Mischungsverhältnisse an Kieselgel reinigt. Nach der Elution werden die Hauptfraktionen kombiniert und unter Vakuum eingeengt. Der Rückstand wird in Dichlormethan aufgenommen, wenn nötig mit n-Pentan versetzt, und im Kühlschrank zur Kristallisation gebracht.

Die folgenden Beispiele erläutern die Erfindung:

Beispiel 1

Herstellung von N-[N'-(2-Chloräthyl)- N'-nitroso-carbamoyl]- glycyl-L-valinmethylester (allgemeine Formel III) $R_1$ = –H $R_3$ = $-CH(CH_3)_2$

N-[N'-(2-Chloräthyl)-N'-nitroso-carbamoyl]-glycin (1 mM) und L-Valinmethylester (1 mM) werden zusammen in 20 ml Dichlormethan gelöst. In dieser Lösung wird unter Rühren Dicyclohexyl-carbodiimid (DCC; 1,1 mM), gelöst in 10 ml Dichlormethan, zugetropft. Das Reaktionsgemisch wird dann noch eine Stunde gerührt und bei Zimmertemperatur über Nacht stehengelassen. Nach Abfiltrieren von N,N'-Dicyclohexylharnstoff wird das Filtrat unter Vakuum eingeengt. Das Rohprodukt wird durch Säulenchromatographie gereinigt (Kieselgel, eluiert mit Äther/Dichlormethan 1:10). Aus den Hauptfraktionen wird das reine Produkt nach Entfernen von Lösungsmittel in Vakuum und durch Umkristallisation aus Dichlormethan/n-Pentan erhalten. Hellgelbe Nadeln, Ausbeute: ca. 70%, $F_p$. 97 bis 98 °C (Zers.); Elementaranalyse: Ber.: C 40,94; H 5,93; N 17,36; Gef.: C 41,09; H 6,00; N 17,16. Die $^1$H-NMR-spektroskopische Untersuchung ergab charakteristische Peaks bei δ = 0,95 ppm (m, $-C(CH_3)_2$); δ = 2,20 ppm (m, –CH=); δ = 3,50 ppm (t, $Cl-CH_2-$); δ = 3,75 ppm (s, $-OCH_3$); δ = 4,20 ppm ($-N-CH_2-CO-$ und $-CH_2-N-NO$); δ = 4,60 ppm (m, –N-CH-C–); δ = 6,70 ppm (d, –NH–); δ = 7,70 ppm (t, –NH–). Das Massenspektrum zeigt kein Signal für ein Molekülion, aber charakteristische Fragmente bei M-107 und M-108, die der Abspaltung von $Cl(CH_2)_2-NNO$ bzw. $Cl(CH_2)_2-N = NOH$ zuzuschreiben sind.

Beispiel 2

Herstellung von N-[N'-(2-Chloräthyl)-N'-nitroso-carbamoyl]- L-valyl-L-valinmethylester (allgemeine Formel III) $R_1 = R_3 = -CH(CH_3)_2$

N-[N'-(2-Chloräthyl)- N'-nitroso-carbamoyl]-L-valin (1 mM) und L-Valinmethylester (1 mM) werden analog wie unter Beispiel 1 behandelt. Das Produkt kristallisiert aus Dichlormethan/n-Pentan als hellgelbe Nadeln, Ausbeute 65%, $F_p$. 125 bis 126 °C (Zers.); Elementaranalyse; Ber.: C 46,09; H 6,91; N 15,36; Gef.: C 46,31; H 7,10, N 15,23. Die $^1$H-NMR-spektroskopische Untersuchung ergab folgende charakteristische Peaks bei: δ = 0,9–1,1 ppm (m, $-C(CH_3)_2$); δ = 2,20 ppm (m, –CH=); δ = 3,50 ppm (t, $Cl-CH_2-$); δ = 3,75 ppm (s, $-OCH_3$); δ = 4,20 ppm (t, $-CH_2-N-NO$); δ = 4,50 ppm (m, –N-CH-CO); δ = 6,60 ppm (d, –NH–); δ = 7,50 ppm (d, –NH–). Das Massenspektrum zeigt einen Molekülpeak bei 364 und Peaks bei m/e = 258 und 257 (M-107 und M-108). Die letzten beiden Peaks entstehen durch Abspaltung von $Cl-CH_2-CH_2-N-NO$ oder $Cl-CH_2-CH_2-N-NOH$, entweder durch einfache Aufspaltung der N-C-Bindung oder durch eine McLafferty Umlagerung.

Beispiel 3

Herstellung von N-[N'-(2-Chloräthyl)- N'-nitroso-carbamoyl]- L-valyl-L- methioninmethylester (allgemeine Formel III) $R_1 = -CH(CH_3)_2$; $R_3 = -CH_2-CH_2-SCH_3$)

N-[N'-(2-Chloräthyl)- N'-nitroso-carbamoyl]-L-Valin (1 mM) und L-Methioninmethylester (1 mM) werden analog wie unter Beispiel 1 behandelt. Gelbliche Nadeln (Dichlormethan/n-Pentan), Ausbeute ca. 70%, $F_p$. 81 bis 82 °C (Zers.). Elementaranalyse: Ber. C 42,37; H 6,35; N 14,12; Gef.: C 42,65; H 6,50; N 14,14. Die $^1$H-NMR-spektroskopische Untersuchung ergab folgende charakteristische Peaks bei: δ = 1,00 ppm (m, $-C(CH_3)_2$); δ = 2,10 ppm ($-CH_2-S-CH_3$); –CH=); δ = 2,55 ppm (t, $-CH_2-$); δ = 3,50 ppm (t, $Cl-CH_2-$); δ = 3,80 ppm (s, $-OCH_3$); δ = 4,20 ppm ($-CH_2-N-NO$); δ = 4,40 ppm (m, –N-CH-CO); δ = 4,80 ppm (m, –N-CH-CO); δ = 6,80 ppm (d, –NH–); δ = 7,50 ppm (d, –NH–).

Das Massenspektrum zeigt ein Molekülpeak bei 396 und Peaks bei m/e = 289 und 288 (M-107 und M-108).

Beispiel 4

Herstellung von N-[N'-(2-Chloräthyl)- N'-nitroso-carbamoyl]- L-isoleucyl-L-leucinmethylester (allgemeine Formel III)

$R_1 = -CH(CH_3)CH_2CH_3$; $R_3 = -CH_2-CH(CH_3)_2$)

N-[N'-(2-Chloräthyl)- N'-nitroso-carbamoyl]-L-isoleucin (1 mM) und L-Leucinmethylester (1 mM) werden analog wie unter Beispiel 1 behandelt. Hellgelbe Nadeln (Dichlormethan/n-Pentan), Ausbeute ca. 60%, $F_p$. 128 bis 129 °C (Zers.). Elementaranalyse: Ber.: C 48,91; H 7,44; N 14,26; Gef.: C 49,03; H 7,70; N 13,96. Die $^1$H-NMR-spektroskopische Untersuchung ergab folgende Peaks bei: δ = 1,00 ppm ($-CH_3$); δ = 1,25–2,00 ppm (–CH=; $-CH_2-$); δ = 3,50 ppm (t, $Cl-CH_2-$); δ = 3,80 ppm (s, $-OCH_3$); δ 4,20 ppm (t, $CH_2-N-NO$); δ = 4,40 ppm (m, –N-CH-CO); δ = 4,70 ppm (m, –N-CH-CO); δ = 6,30 ppm (d, –NH–); δ = 7,45 ppm (d, –NH–). Das Massenspektrum zeigt ein Molekül-Peak bei 392 und Peaks von m/e = 286,285 (M-107, M-108).

Beispiel 5

Herstellung von N-[N'-(2-Chloräthyl)- N'-nitroso-carbamoyl]- L-phenylalanyl-L- valinmethylester (III, $R_1 = -CH_2-C_6H_5$; $R_3 = -CH(CH_3)_2$)

N-[N'-(2-Chloräthyl)- N'-nitroso-carbamoyl]-L- phenylalanin (1 mM) und L-Valinmethylester (1 mM) werden analog wie unter Beispiel 1 behandelt. Gelbe Nadeln (Dichlormethan/n-Pentan), Ausbeute ca. 60%, $F_p$. 107 bis 108 °C.

Elementaranalyse: Ber.: C 52,36; H 6,10; N 13,57; Gef.: C 52,54; H 6,21; N 13,28.

Die $^1$H-NMR-spektroskopische Untersuchung ergab folgende Peaks bei: δ = 0,85 ppm (m, $-C(CH_3)_2$); δ = 2,10 ppm (m, –CH=); δ = 3,15 ppm (d, $-CH_2$-ph); δ = 3,40 ppm (t, $Cl-CH_2-$); δ = 3,70 ppm (s, $-OCH_3$); δ = 4,10 ppm (t, $-CH_2-N-NO$); δ = 4,45 und

4,80 ppm (m, –N–CH–CO); δ = 6,35 ppm (d, –NH–); δ = 7,25 ppm (–$C_6H_5$); δ = 7,55 ppm (d, –NH–). Das Massenspektrum zeigt einen Molekül-Peak bei 412 und Peaks bei m/e = 305, 304 (M-107, M-108).

Beispiel 6

Herstellung von N-[N'-(2-Chloräthyl)-N'-nitroso-carbamoyl]-L-phenylalanyl-L-leucin-methylester (allgemeine Formel III)
$R_1$ = –$CH_2$–$C_6H_5$; $R_3$ = –$CH_2$–$CH(CH_3)_2$)

N-[N'-(2-Chloräthyl)-N'-nitroso-carbamoyl]-L-phenylalanin (1 mM) und L-Leucinmethylester (1 mM) werden analog wie unter Beispiel 1 behandelt. Hellgelbe Nadeln (Dichlormethan/n-Pentan), Ausbeute ca. 60%, $F_p$. 96 bis 96,5 °C.

Elementaranalyse: Ber.: C 53,46; H 6,38; N 13,12; Gef.: C 53,29; H 6,34; N 12,92.

Die $^1$H-NMR-spektroskopische Untersuchung ergab charakteristische Peaks bei: δ = 0,90 ppm (–$C(CH_3)_2$); δ = 1,50 ppm (m, –$CH_2$–CH=); δ = 3,15 ppm (d, –$CH_2$–ph); δ = 3,45 ppm (t, Cl–$CH_2$–); δ = 3,75 ppm (s, –$OCH_3$); δ = 4,10 ppm (t, –$CH_2$–N–NO); δ = 4,55 ppm (m, –N–CH–CO); δ = 4,80 ppm (m, –N–CH–CO); δ = 6,20 ppm (d, –NH–); δ = 7,30 ppm (–$C_6H_5$); δ = 7,50 ppm (d, –NH–).

Das Massenspektrum zeigt Molekül-Peaks bei 426 und Peaks bei m/e = 319, 318 (M-107, M-108).

Beispiel 7

Herstellung von N-[N'-(2-Chloräthyl)-N'nitroso-carbamoyl]-L-phenylalanyl-L-phenylalanin-methylester (allgemeine Formel III)
$R_1$ = $R_3$ = –$CH_2$–$C_6H_5$).

N-[N'-(2-Chloräthyl)-N'-nitroso-carbamoyl]-L-phenylalanin (1 mM) und L-Phenylalaninmethylester (1 mM) werden analog wie unter Beispiel 1 behandelt. Hellgelbe Nadeln (Dichlormethan/n-Pentan), Ausbeute ca. 60%, $F_p$. 116 bis 117 °C.

Elementaranalyse: Ber.: C 57,33; H 5,47; N 12,16; Gef.: C 57,22; H 5,23; N 12,12.

Die $^1$H-NMR-spektroskopische Untersuchung ergab folgende Peaks bei: δ = 3,10 ppm ($CH_2$–ph); δ = 3,40 ppm (t, Cl–$CH_2$–); δ = 3,70 ppm (s, –$OCH_3$); δ = 4,10 ppm (t, –$CH_2$–N–NO); δ = 4,75 ppm (–N–CH–CO); δ = 6,10 ppm (d, –NH–); δ = 7,20 ppm (–$C_6H_5$); δ 7,40 ppm (d, –NH–).

Das Massenspektrum zeigt Molekül-Peaks bei 460 und Peaks bei m/e = 353, 352 (M-107, M-108).

Beispiel 8

Herstellung von N-[N'-(2-Chloräthyl)-N'-nitroso-carbamoyl]-L-phenylalanyl-L-methionin-methylester (allgemeine Formel III)
$R_1$ = –$CH_2$–$C_6H_5$; $R_3$= $CH_2$–$CH_2$–$SCH_3$)

N-[N'-(2-Chloräthyl)-N'-nitroso-carbamoyl]-L-phenylalanin (1 mM) und L-Methioninmethylester (1 mM) werden analog wie unter Beispiel 1 behandelt. Hellgelbe Nadeln (Dichlormethan/n-Pentan), Ausbeute ca. 70%, $F_p$. 74 bis 75 °C.

Elementaranalyse: Ber.: C 48,59; H 5,66; N 12,59; Gef.: C 48,69; H 5,67; N 12,41.

Die $^1$H-NMR-spektroskopische Untersuchung ergab folgende charakteristische Peaks bei: δ = 2,05 ppm (–$CH_2$–S–$CH_3$); δ = 2,45 ppm (–$CH_2$–); δ = 3,20 ppm (d, –$CH_2$–ph); δ = 3,45 ppm (t, Cl–$CH_2$–); δ = 3,75 ppm (s, –$OCH_3$); δ = 4,10 ppm (t, –$CH_2$–N–NO); δ = 4,65 ppm (m, –N–CH–CO); δ = 4,80 ppm (m, –N–CH–CO); δ = 6,50 ppm (d, –NH–); δ = 7,25 ppm (–$C_6H_5$); δ = 7,50 ppm (d, –NH–).

Das Massenspektrum zeigt einen Molekül-Peak bei 444 und Peaks bei m/e = 337, 336 (M-107, M-108).

Beispiel 9

Herstellung von N-[N'-(2-Chloräthyl)-N'-nitroso-carbamoyl]-L-methionyl-L-phenylalanin-methylester (allgemeine Formel III)
$R_1$ = –$CH_2$–$CH_2$–$SCH_3$; $R_3$ = $CH_2$–$C_6H_5$).

N-[N'-(2-Chloräthyl)-N'-nitroso-carbamoyl]-L-methionin (1 mM) und L-Phenylalaninmethylester (1 mM) werden analog wie unter Beispiel 1 behandelt. Hellgelbe Nadeln (Dichlormethan/n-Pentan), Ausbeute ca. 70%, $F_p$. 70 bis 71 °C.

Elementaranalyse: Ber.: C 48,59; H 5,66; N 12,59; Gef.: C 48,64; H 5,68; N 12,36.

Die $^1$H-NMR-spektroskopische Untersuchung ergab folgende Peaks bei: δ = 2,05–2,20 ppm (–$CH_2$–S–$CH_3$); δ 2,65 ppm (t, –$CH_2$–); δ = 3,10 ppm (d, –$CH_2$–pH); δ = 3,45 ppm (t, Cl–$CH_2$–); δ = 3,75 ppm (s, –$OCH_3$); δ = 4,10 ppm (t, –$CH_2$–N–NO); δ = 4,70–4,90 ppm (m, –N–CH–CO); δ = 6,80 ppm (d, –NH–); δ = 7,20 ppm (m, –$C_6H_5$); δ = 7,65 ppm (d, –NH–). Das Massenspektrum zeigt einen Molekül-Peak bei 444 und Peaks bei m/e = 337, 336 (M-107, M-108).

## Patentansprüche

1. N-[N'-(2-Chloräthyl)-N'-nitroso-carbamoyl]-oligopeptidester und -amide der allgemeinen Formel

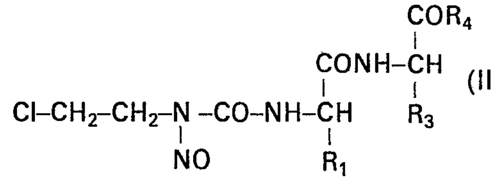 (III)

worin

$R_1$ = $R_3$ = –H, –$CH_3$, –$CH(CH_3)_2$, –$CH_2$–$CH(CH_3)_2$,

–$C_2H_5$, –$CH(CH_3)C_2H_5$, –$CH_2$– 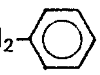 , –$CH_2OH$,

–$CH(OH)CH_3$, –$CH_2$–$CH_2$–S–$CH_3$,

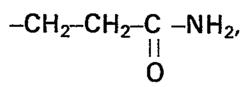 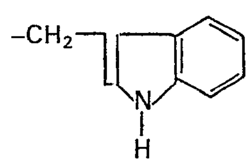

$R_4$ = –$OCH_3$, –$NH_2$, NH–$R_4'$ wobei $R_4'$ einen Oligopeptidrest darstellt, dessen endständige

Carboxylgruppe ihrerseits als Methylester oder Amidgruppe verschlossen ist.

2. N-[N'-(2-Chloräthyl)-N'-nitroso-carbamoyl]-glycyl-L-valinmethylester.
3. N-[N'-(2-Chloräthyl)-N'-nitroso-carbamoyl]-L-valyl-L-valinmethylester.
4. N-[N'-(2-Chloräthyl)-N'-nitroso-carbamoyl]-L-valyl-L-methioninmethylester.
5. N-[N'-(2-Chloräthyl)-N'-nitroso-carbamoyl]-L-isoleucyl-L-leucinmethylester.
6. N-[N'-(2-Chloräthyl)-N'-nitroso-carbamoyl]-L-phenylalanyl-L-valinmethylester.
7. N-[N'-(2-Chloräthyl)-N'-nitroso-carbamoyl]-L-phenylalanyl-L-leucinmethylester.
8. N-[N'-(2-Chloräthyl)-N'-nitroso-carbamoyl]-L-phenylalanyl-L-phenylalaninmethylester.
9. N-[N'-(2-Chloräthyl)-N'-nitroso-carbamoyl]-L-phenylalanyl-L-methioninmethylester.
10. N-[N'-(2-Chloräthyl)-N'-nitroso-carbamoyl]-L-methionyl-L-phenylalaninmethylester.
11. Verfahren zur Herstellung von N-[N'-(2-Chloräthyl)- N'-nitroso-carbamoyl]- oligopeptidestern oder -amiden nach Anspruch 1, dadurch gekennzeichnet, dass N-[N'-(2-Chloräthyl)- N'-nitroso-carbamoyl]- aminosäuren oder -oligopeptide mit einem Ester oder Amid einer zweiten Aminosäure oder eines Oligopeptids die gleich den ersten oder verschieden sein können unter Einwirkung eines geeigneten basischen Peptidkatalysators in der Kälte zu N-[N'-(2-Chloräthyl)- N'-nitroso-carbamoyl]-oligopeptidestern oder -amiden umgesetzt werden.
12. Verfahren nach Anspruch 11, dadurch gekennzeichnet, dass die als Ausgangsmaterial verwendetenN-[N'-(2-Chloräthyl)-N'-nitroso-carbamoyl]- aminosäuren oder -oligopeptide aus N-(2-Chloräthyl)- N-nitroso-carbamoylazid und Aminosäure bzw. Oligopeptid in an sich bekannter Weise in einem geeigneten Lösungsmittel, insbesondere Dichlormethan hergestellt werden und diese Reaktionslösung ohne Isolierung für die Umsetzung mit dem Aminosäureester oder -amid- oder entsprechenden Oligopeptid eingesetzt wird.
13. Verfahren nach Anspruch 11 oder 12, dadurch gekennzeichnet, dass als basischer Peptidkatalysator Dicyclohexylcarbodiimid verwendet wird.

**Revendications**

1. Esters et amides N-[N'-(2-chloréthyl)-N'-nitroso-carbamoyl]- oligopeptidiques de formule générale

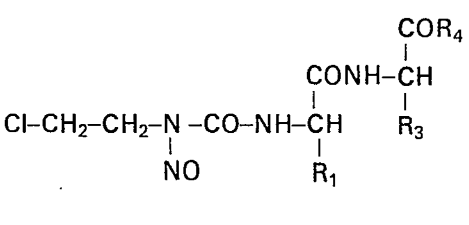

(III)

où

$R_1 = R_3 = -H, -CH_3, -CH(CH_3)_2, -CH_2-CH(CH_3)_2,$

$-C_2H_5, -CH(CH_3)C_2H_5, -CH_2-$ 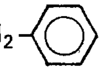 $, -CH_2OH,$

$-CH(OH)CH_3, -CH_2-CH_2-S-CH_3,$

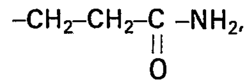

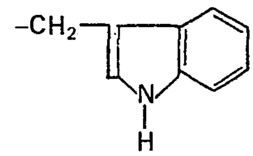

$R_4 = -OCH_3, -NH_2, -NH-R_4'$, où $R_4'$ est un résidu oligopeptidique, dont le groupe carboxyle terminal est, pour sa part, bloqué sous la forme d'un ester méthylique ou d'un groupe amide.

2. Ester méthylique de la N-[N'- (2-chloréthyl)-N'-nitroso-carbamoyl]-glycyl-L-valine.
3. Ester méthylique de la N-[N'- (2-chloréthyl)-N'-nitroso-carbamoyl]-L-valyl-L-valine.
4. Ester méthylique de la N-[N'- (2-chloréthyl) N'-nitroso-carbamoyl]-L-valyl-L-méthionine.
5. Ester méthylique de la N-[N'- (2-chloréthyl)-N'-nitroso-carbamoyl]-L-isoleucyl-L-leucine.
6. Ester méthylique de la N-[N'- (2-chloréthyl)-N'-nitroso-carbamoyl]-L-phénylalanyl-L-valine.
7. Ester méthylique de la N-[N'- (2-chloréthyl)-N'-nitroso-carbamoyl]-L-phénylalanyl-L-leucine.
8. Ester méthylique de la N-[N'- (2-chloréthyl)-N'-nitroso-carbamoyl]-L-phénylalanyl-L-phénylalanine.
9. Ester méthylique de la N-[N'- (2-chloréthyl)-N'-nitroso-carbamoyl]-L-phénylalanyl-L-méthionine.
10. Ester méthylique de la N-[N'- (2-chlorethyl)-N'-nitroso-carbamoyl]-L-méthionyl-L-phénylalanine.
11. Procédé pour la préparation d'esters ou amides N-[N'-(2-chloréthyl)- N'-nitroso-carbamoyl]- oligopeptidiques selon la revendication 1, caractérisé en ce qu'on fait réagir des acides N-[N'- (2-chloréthyl)- N'-nitroso-carbamoyl]-aminés ou leurs oligopeptides sur un ester ou un amide d'un deuxième acide aminé ou d'un oligopeptide, qui peuvent être identiques ou différents des premiers, sous l'action d'un catalyseur peptidique basique convenable, à froid, pour donner des esters ou amides N-[N'-(2-chloréthyl)- N'-nitroso-carbamoyl]- oligopeptidiques.
12. Procédé selon la revendication 1, caractérisé en ce que les acides N-[N'- (2-chloréthyl)-N'-nitroso-carbamoyl]- aminés on leurs oligopeptides utilisé en tant que matière de départ sont préparés à partir de N-(2-chloréthyl)-N- nitroso-carbamoylazide et respectivement d'un acide aminé ou d'un oligopeptide, d'une manière connue en soi, dans un solvant approprié, en particulier le dichlorométhane, et que cette solution de réaction est utilisée sans isolation pour la réaction sur l'ester ou l'amide de l'acide aminé ou l'oligopeptide correspondant.
13. Procédé selon la revendication 11 ou 12,

caractérisé en ce qu'on utilise en tant que catalyseur peptidique basique le dicyclohexylcarbodiimide.

## Claims

1. N-[N'-(2-Chloroethyl)-N'-nitroso-carbamoyl]- oligopeptide esters and amides of the general formula

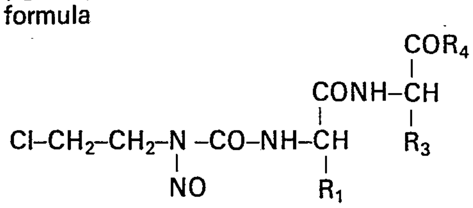

(III)

wherein

$R_1 = R_3 = -H, -CH_3, -CH(CH_3)_2, -CH_2-CH(CH_3)_2,$

$-C_2H_5, -CH(CH_3)C_2H_5, -CH_2-$ 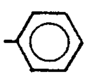 , $-CH_2OH$,

$-CH(OH)CH_3, -CH_2-CH_2-S-CH_3,$

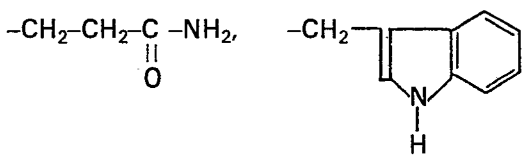

and

$R_4 = -OCH_3$, $-NH_2$, or $NH-R_4$ wherein $R_4$ represents an oligopeptide radical of which the terminal carboxyl group, is, in its turn, closed (sic) as a methyl ester or amide groupe.

2. N-[N'-(2-Chloroethyl)-N'-nitroso-carbamoyl]-glycyl-L-valine methyl ester.

3. N-[N'-(2-Chloroethyl)-N'-nitroso-carbamoyl]-L-valyl-L-valine methyl ester.

4. N-[N'-(2-Chloroethyl)-N'-nitroso-carbamoyl]-L-valyl-L-methionine-methyl ester.

5. N-[N'-(2-Chloroethyl)-N'-nitroso-carbamoyl]-L-isoleucyl-L-leucine methyl ester.

6. N-[N'-(2-Chloroethyl)-N'-nitroso-carbamoyl]-L-phenylalanyl-L-valine methyl ester.

7. N-[N'-(2-Chloroethyl)-N'-nitroso-carbamoyl]-L-phenylalanyl-L-leucine methyl ester.

8. N-[N'-(2-Chloroethyl)-N'-nitroso-carbamoyl]-L-phenylalanyl-L-phenylalanine methyl ester.

9. N-[N'-(2-Chloroethyl)-N'-nitroso-carbamoyl]-L-phenylalanyl-L-methionine methyl ester.

10. N-[N'-(2-Chloroethyl)-N'-nitroso-carbamoyl]-L-methionyl-L-phenylalanine methyl ester.

11. Process for the preparation of N-[N'-(2-Chloroethyl)-N'-nitroso-carbamoyl]-oligopeptide esters or amides according to Claim 1, characterised in that N-[N'-(chloroethyl)-N'-nitroso-carbamoyl]- amino acids (sic) or-oligopeptides are reacted with an ester or amide of a second amino acid or of an oligopeptide, which may be identical to the first or different therefrom, under the action of a suitable basic peptide catalyst, in the cold, to give N-[N'-(2-chloroethyl)-N'-nitroso-carbamoyl]-oligopeptide esters or amides.

12. Process according to Claim 11, characterised in that the N-[N'-(2-chloroethyl)-N'-nitroso-carbamoyl]- amino acids or -oligopeptides used as starting material are prepared from N-(2-chloroethyl)-N-nitroso-carbamoyl azide and amino acid or oligopeptide in a manner known per se, in a suitable solvent, especially methylene chloride, and this reaction solution is employed, without isolation, for the reaction with the amino acid ester or amide or corresponding oligopeptide.

13. Process according to Claim 11 or 12, characterised in that dicyclohexylcarbodiimide is used as the basic peptide catalyst.